# EUROPEAN PATENT SPECIFICATION

(11) **EP 3 445 849 B1**
(45) Date of publication and mention of the grant of the patent: **05.06.2024**
(21) Application number: 17786683.7
(22) Date of filing: 21.04.2017
(51) Int. Cl.: C12N 15/00, C07K 14/54, A61K 35/17, A61K 35/30, A61K 9/00, A61K 38/17, A61K 38/20, A61P 25/00, C07K 14/715, C12N 7/00

(54) **METHODS AND COMPOSITIONS TO ENHANCE THE ANTI-INFLAMMATORY EFFECTS OF INTERLEUKIN 10**
VERFAHREN UND ZUSAMMENSETZUNGEN ZUR VERBESSERUNG DER ENTZÜNDUNGSHEMMENDEN WIRKUNG VON INTERLEUKIN 10
PROCÉDÉS ET COMPOSITIONS DESTINÉS À AMÉLIORER LES EFFETS ANTI-INFLAMMATOIRES DE L'INTERLEUKINE 10

(30) Priority: 22.04.2016 US 201662326082 P
(43) Date of publication of application: 27.02.2019
(73) Proprietor: Xalud Therapeutics, Inc., Charlottesville, VA 22902 (US); The Regents of the University of Colorado, a body corporate, Denver, CO 80203 (US)
(72) Inventor: FORSAYETH, John, Berkeley, CA 94704 (US); CHAVEZ, Raymond, Berkeley, CA 94704 (US); WATKINS, Linda, Denver, CO 80203 (US); GRACE, Peter, Denver, CO 80203 (US)
(74) Representative: Mewburn Ellis LLP
(86) International application number: PCT/US2017/028755
(87) International publication number: WO 2017/184933

(56) References cited:
- WO-A1-2015/009955
- US-A- 5 716 804
- US-A1- 2009 035 256
- US-A1- 2012 058 102
- N. TAMASSIA ET AL: "Circulating neutrophils of septic patients constitutively express IL-10R1 and are promptly responsive to IL-10", INTERNATIONAL IMMUNOLOGY, vol. 20, no. 4, 27 February 2008 (2008-02-27), pages 535-541, XP055611408, ISSN: 0953-8178, DOI: 10.1093/intimm/dxn015
- SUNG IL YOON ET AL: "Epstein-Barr Virus IL-10 Engages IL-10R1 by a Two-step Mechanism Leading to Altered Signaling Properties", JOURNAL OF BIOLOGICAL CHEMISTRY, vol. 287, no. 32, 3 August 2012 (2012-08-03), pages 26586-26595, XP055611410, US ISSN: 0021-9258, DOI: 10.1074/jbc.M112.376707
- Mark R. Walter: "The Molecular Basis of IL-10 Function: from Receptor Structure to the Onset of Signaling" In: "Current topics in microbiology and immunology", 1 January 2014 (2014-01-01), Springer Berlin Heidelberg, Berlin, Heidelberg, XP055529329, ISSN: 0070-217X ISBN: 978-3-540-92165-3 vol. 380, pages 191-212, DOI: 10.1007/978-3-662-43492-5_9, * paragraph [03.1] - paragraph [03.3] *
- LOGAN BROCK J: "Sex Differences in the Efficacy of Combined IL-10 and IL-10R1 Gene Therapy for Neuropathic Pain in Mice", THESIS, UNIVERSITY OF COLORADO, BOULDER, COLORADO, USA, [Online] 12 May 2016 (2016-05-12), pages 1-55, XP009515091, Retrieved from the Internet: URL:https://scholar.colorado.edu/honr_thes es/1131/>
- CREPALDI ET AL.: 'Up-Regulation of IL-10R1 Expression Is Required to Render Human Neutrophils Fully Responsive to IL-10' THE JOURNAL OF IMMUNOLOGY vol. 167, 2001, pages 2312 - 2322, XP055431545

## Description

### CROSS REFERNCE TO RELATED A PPLICATIONS

### FIELD OF THE INVENTION

This invention relates to methods and compositions for attenuating deactivation of inflammatory signaling by expressing, in addition to an interleukin 10 (IL-10) peptide, an IL-10 receptor type 1 (IL-10R1) peptide. The methods have use in treating a variety of conditions including but not limited to neuropathic pain; symptoms associated with multiple sclerosis, spinal cord injury, ALS, neuroinflammation, arthritis and other diseases of the joint, as well as autoimmune diseases.

### BACKGROUND OF THE INVENTION

In the following discussion certain articles and methods will be described for background and introductory purposes. Nothing contained herein is to be construed as an "admission" of prior art. Applicant expressly reserves the right to demonstrate, where appropriate, that the articles and methods referenced herein do not constitute prior art under applicable statutory provisions.

Inflammation is associated with the release of pain-enhancing pro-inflammatory cytokines and radicals, such as H₂O₂ and NO. In order to limit inflammation and damage, anti-inflammatory cytokines such as interleukin-10 are also released; for example, in inflamed tissues, IL-10 is elevated to concentrations sufficient to cap excessive inflammation. Previous studies have shown that plasmid-directed expression of human interleukin-10 (hIL-10) is anti-allodynic when injected intrathecally in rodent models of neuropathic pain (see Watkins, USSN 14/066,581), and multiple sclerosis (MS) (see Watkins, et al., USSN 14/370,724), as well as intra-articularly in large animals with either naturally occurring (dogs) or surgically-induced (horse) osteoarthritis (OA) (see Chavez, et al., USSN 14/905,915). IND-enabling studies conducted over the past several years have provided convincing evidence that this approach to the treatment of chronic pain is likely to be both safe and effective in humans. In the course of these studies, however, it has become clear that IL-10 evinces maximum signaling efficiency when present at concentrations below 1 ng/ml, and that increasing concentrations of IL-10 paradoxically result in down-regulation of IL-10. There is a need in the art for methods and compositions that can overcome the down-regulation of IL-10 signaling to achieve a more powerful IL-10-mediated suppression of inflammation that is not dose-dependent. The present invention addresses this need.

### SUMMARY OF THE INVENTION

The invention is defined in the appended claims and relates to methods and compositions for overcoming dose-dependent down-regulation of IL-10 by expressing, in addition to an interleukin 10 (IL-10) peptide, an IL-10 receptor type 1 (IL-10R1) peptide (herein an "IL-10/IL-10R1 expression vector") in an antigen-presenting cell. The methods have use in treating chronic or neuropathic pain; symptoms and physiological damage associated with multiple sclerosis, spinal cord injury, neuroinflammation, ALS, and arthritis and other diseases of the joint; as well as autoimmune diseases.

Thus, the disclosure provides in one embodiment a method for treating inflammation in a subject comprising expression from one or more bacterial, viral, phage, cosmid or artificial chromosome vectors interleukin-10 (IL-10) peptide and interleukin 10 type 1 receptor (IL-10R1) peptide in antigen-presenting cells in the subject. In some aspects of this embodiment, the IL-10 peptide expressed in the antigen-presenting cells comprises a mutation in a hinge region of the IL-10 peptide, and in some aspects, the IL-10 peptide comprises a mutation where a phenylalanine at position 129 of an IL-10 wildtype sequence has been replaced with serine, threonine, alanine or cysteine. In some preferred aspects, the phenylalanine at position 129 of a wildtype sequence has been replaced with serine.

In some embodiments, the IL-10 and IL-10R1 peptides are expressed from a single vector, and in some aspects, the vector is a viral vector, the viral vector is an adeno-associated virus vector, or the viral vector is a lentivirus vector. In some aspects, the IL-10 and IL-10R1 coding sequences are transcribed as a single mRNA, and the vector comprises a coding sequence for an internal ribosome entry site between the IL-10 and the IL-10R1 coding sequences or a coding sequence for a self-cleaving 2a peptide between the IL-10 and IL-10R1 coding sequences.

In some embodiments, the inflammation is caused by neuropathic or chronic pain and the one or more vectors are delivered by intrathecal injection. In yet other embodiments, the inflammation is caused by MS and the one or more vectors are delivered by intrathecal injection. In yet other embodiments, the inflammation is caused by an autoimmune disease and the one or more vectors are delivered by intrathecal injection. In further embodiments, the inflammation is located in a joint and the one or more vectors are delivered by intra-articular injection. In yet other embodiments, the inflammation is neuroinflammation.

In some embodiments, the antigen-presenting cells are selected from the group of monoblasts, monocytes, astrocytes, oligodendrocytes, microglia, macrophages, B cells, dendritic cells, foam cells, lymphoblasts, and B lymphocytes. In some aspects of this embodiment, the antigen-presenting cells are removed from a subject to be treated, transduced with the one or more vectors *in vitro,* and administered back to the subject; however, in some aspects, the antigen-presenting cells are stably transformed with the one or more vectors and are maintained in culture.

Yet other embodiments of the invention provide a single viral or bacterial expression vector comprising the coding regions for interleukin 10 (IL-10) and interleukin 10 type 1 receptor (IL-10R1). In some aspects of this embodiment, the vector is a viral vector comprising a single promoter driving the transcription of the IL-10 and IL-10R1 peptides, and further comprises a self-cleaving 2a peptide positioned between the coding region of the IL-10 peptide and the coding region of the IL-10R1 peptide.

These and other aspects and uses of the invention will be described in the detailed description.

### BRIEF DESCRIPTION OF THE FIGURES

Figure 1 is a plot of absolute threshold (g) versus days after injection for results obtained from injecting rats with AAV-9-hIL-10 in a chronic constriction injury model of neuropathic pain.
Figure 2 is a plot of absolute threshold (g) versus days post injection for results obtained from injecting rats with a 1:1 mixture of AAV9-hIL-10 and AAV9-hIL10R1 in a chronic constriction injury model of neuropathic pain.
Figure 3 is a plot of motor score versus time post onset of motor symptoms (days) for results obtained from intrathecally injecting rats with IL-10 encapsulated in PLGA microparticles (circles) in relapsing-remitting EAE rats. Motor scores: 0 = normal; 1 = tail tip paralysis; 2 = full tail paralysis; 3 = hind leg weak; 4 = hind-leg paralysis; 5 = full hind-leg paralysis; 6 - partial foreleg paralysis. N = 6 per group.
Figure 4 shows the results of microinjection of IL-10 plasmid into the brains of normal mice and an inflammatory mouse model of Down Syndrome (Dp16).

### DETAILED DESCRIPTION OF THE INVENTION

### Definitions

Unless defined otherwise, all technical and scientific terms used herein have the same meaning as commonly understood by one of ordinary skill in the art to which this invention belongs. All publications, published patent applications and patents mentioned herein are incorporated by reference in their entirety for the purpose of describing and disclosing devices, animal models, formulations and methodologies that may be used in connection with the presently described invention.

As used herein, "antigen-presenting cell" refers to any one of various cells that display antigen complexed with major histocompatibility class II complexes (MHCs) on their surfaces (antigen presentation) and that express IL-10 receptor type 2 (IL-10R2). Antigen-presenting cells of the present invention include but are not limited to astrocytes, oligodendrocytes, microglia, macrophages, B cells, dendritic cells and precursors thereof.

The term "anti-inflammatory" as used herein refers to decreasing the action or production of one or more pro-inflammatory cytokines produced by nerves, neurons, glial cells, endothelial cells, fibroblasts, muscle, immune cells or other cell types.

The term "anti-inflammatory cytokine" as used herein refers to a protein that decreases the action or production of one or more pro-inflammatory cytokines or proteins produced by nerves, neurons, glial cells, endothelial cells, fibroblasts, muscle, immune cells or other cell types. Inflammatory cytokines and proteins include, without limitation, interleukin-1 beta (IL-1β), tumor necrosis factor-alpha (TNF-α), interleukin-6 (IL-6), inducible nitric oxide synthetase (iNOS) and the like. The anti-inflammatory cytokine of interest in the present invention is interleukin-10 (IL-10), full-length molecules and fragments of IL-10, as well as modified IL-10 peptides including those with deletions, additions and substitutions (either conservative or non-conservative in nature), to the native sequence so long as the anti-inflammatory cytokine is therapeutically effective. Modifications may be deliberate, as through site-directed mutagenesis, or may be accidental, such as through mutations of hosts which produce the proteins or errors due to PCR amplification. Accordingly, active proteins are typically substantially homologous to the parent sequence, *e.g.,* proteins are typically more than 70%, identical to the parent sequence.

The term "autoimmune disease" as used herein, refers to a pathologic state arising from an abnormal immune response of the body against substances and tissues normally present in the body.

"Chronic pain" as used herein refers to pain that persists longer than the temporal course of natural healing associated with a particular type of injury or disease process.

The term DNA "control sequences" refers collectively to promoter sequences, polyadenylation signals, transcription termination sequences, upstream regulatory domains, origins of replication, internal ribosome entry sites, enhancers, and the like, which collectively provide for the replication, transcription and translation of a coding sequence in a recipient cell. Not all of these types of control sequences need to be present so long as the selected coding sequence is capable of being replicated, transcribed and translated in an appropriate host cell.

A "coding sequence" of, e.g., IL-10 or IL-10R1 or a sequence that "encodes" IL-10 or IL-10R1 is a nucleic acid molecule that is transcribed (in the case of DNA) and translated (in the case of mRNA) into a polypeptide *in vivo* when placed under the control of appropriate control sequences. The boundaries of the coding sequence are determined by nucleotides corresponding to a start codon at the amino terminus and nucleotides corresponding to a translation stop codon at the carboxy-terminus.

The terms "effective amount" or "therapeutically effective amount" of a therapeutic microparticle composition used in the methods of the invention refer to a nontoxic but sufficient amount of the therapeutic microparticle composition to provide the desired response, such as a decrease in pain, a decrease in inflammation, relief from symptoms caused by inflammatory diseases and/or preventing progression of physiological damage due to inflammatory diseases, and/or relief from symptoms caused by, e.g., MS, chronic pain, joint inflammation, neuroinflammation, and autoimmune diseases. The exact amount of the therapeutic anti-inflammatory composition of the present invention required will vary from subject to subject, depending on the species, age, and general condition of the subject, the severity of the condition being treated, the particular IL-10/IL-10R1 expression vector to be delivered, mode of administration, and the like. Dosage parameters for the present methods are provided herein; however, optimization of an appropriate "effective" amount in any individual case may be determined by one of ordinary skill in the art via the methods set forth herein and routine experimentation.

The term "excipient" refers to an inert substance added to a pharmaceutical composition of the invention to further facilitate administration of the therapeutic microparticle composition. Examples, without limitation, of excipients include saline, calcium carbonate, calcium phosphate, various sugars and types of starch, cellulose derivatives, gelatin, hyaluronic acid optionally formulated with a surfactant, Pluronic F-68, vegetable oils and polyethylene glycols.

The term *"in vitro"* refers to events that occur in an artificial environment, e.g., in a test tube or reaction vessel, in cell culture, etc., rather than within an organism.

The term "joint" refers to an anatomical structure where two bones meet, including the ligaments that connect the bones to one another, the tendons that attach muscles to the bones, the joint capsule, bursa and synovium. Joints that can be treated with the methods herein include fixed, hinge, pivot or ball-and-socket joints.

The term "joint inflammation" or "joint pain" refers to all types of arthritis caused by inflammation, where rheumatoid arthritis, osteoarthritis are the most common, as well as other conditions caused by inflammation of the joints including tendonitis, bursitis, inflammation of the ligament, synovitis, gout, and systemic lupus erythematosus.

As used herein, the term "multiple sclerosis" or "MS", refers to a progressive, neurodegenerative disease of the central nervous system, which occurs most often in a relapsing/remitting form in which a period of demyelination is followed by a period of functional recovery. The recovery stage involves remyelination via migration and maturation of oligodendrocyte precursor cells or oligodendrocyte progenitor cells. However, as the disease progresses, remyelination fails with progressive loss of function. Possible explanations for remyelination failure of intact axons include defects in oligodendrocyte precursor cell recruitment to the site of demyelination or defects in oligodendrocyte precursor cell differentiation into myelinating oligodendrocytes. Although studies indicate that both aspects of oligodendrocyte precursor cell biology are altered in MS, the molecular mechanisms that orchestrate these processes within the adult central nervous system are incompletely understood but are known to include inflammation.

The term "nuclear targeting sequence" refers to a nucleic acid sequence which functions to improve the expression efficiency of the anti-inflammatory cytokine in a cell.

"Operably linked" refers to an arrangement of elements where the components so described are configured so as to perform their usual function. Thus, control sequences operably linked to a coding sequence are capable of effecting the expression of the coding sequence. Control sequences need not be contiguous with the coding sequence so long as they function to direct the expression thereof. Thus, for example, intervening untranslated yet transcribed sequences can be present between a promoter sequence and the coding sequence and the promoter sequence can still be considered "operably linked" to the coding sequence.

The term "promoter" is used herein in its ordinary sense to refer to a nucleotide region comprising a DNA regulatory sequence, wherein the regulatory sequence is derived from a gene that is capable of binding RNA polymerase and initiating transcription of a downstream (3'-direction) coding sequence. Transcription promoters can include "inducible promoters" (where expression of a polynucleotide sequence operably linked to the promoter is induced by an analyte, cofactor, regulatory protein, etc.), "repressible promoters" (where expression of a polynucleotide sequence operably linked to the promoter is induced by an analyte, cofactor, regulatory protein, etc.), and "constitutive promoters".

For the purpose of describing the relative position of nucleotide sequences in a particular nucleic acid molecule throughout the instant application, such as when a particular nucleotide sequence is described as being situated "upstream," "downstream," "3 prime (3')" or "5 prime (5')" relative to another sequence, it is to be understood that it is the position of the sequences in the "sense" or "coding" strand of a DNA molecule that is being referred to as is conventional in the art.

The term "research tool" as used herein refers to any methods of the invention that uses the therapeutic microparticle composition for scientific inquiry, either academic or commercial in nature, including the development of other pharmaceutical and/or biological therapeutics. The research tools of the invention are not intended to be therapeutic or to be subject to regulatory approval; rather, the research tools of the invention are intended to facilitate research and aid in such development activities, including any activities performed with the intention to produce information to support a regulatory submission.

As used herein the term "selectable marker" refers to a gene introduced into a cell, particularly in the context of this invention into cells in culture that confers a trait suitable for artificial selection. General use selectable markers are well-known to those of ordinary skill in the art.

The terms "subject", "individual" or "patient" may be used interchangeably herein and refer to a vertebrate, preferably a mammal.

The term "therapeutic composition" or "therapeutic anti-inflammatory composition" as used herein refers to an IL-10/IL-10R1 expression vector composition that has the ability to decrease the concentration and/or inflammatory action of inflammatory cytokines. The therapeutic compositions of the present invention are useful in treating, e.g., MS, chronic pain, joint inflammation, neuroinflammation, and autoimmune diseases as measured in any of the known animal models or by assessment performed in humans.

"Treatment" or "treating" inflammation includes: (1) decreasing inflammation or causing the inflammation to occur with less intensity in a subject that may be predisposed to inflammation but does not yet experience or display symptoms, (2) inhibiting inflammation, *i.e.,* arresting the development of or reversing symptoms or physiological damage caused by inflammation, or (3) decreasing or reversing the physiological damage resulting from inflammation.

A "vector" is a replicon, such as plasmid, phage, viral construct, cosmid, bacterial artificial chromosome, human-derived artificial chromosome or yeast artificial chromosome to which another heterologous DNA segment may be inserted. Vectors herein are used to transduce and express the IL-10 and IL-10R1 peptides.

The practice of the techniques described herein may employ, unless otherwise indicated, conventional techniques and descriptions of organic chemistry, polymer technology, molecular biology (including recombinant techniques), cell biology, biochemistry, and sequencing technology, which are within the skill of those who practice in the art. Specific illustrations of suitable techniques can be had by reference to the examples herein. However, other equivalent conventional procedures can, of course, also be used. Such conventional techniques and descriptions can be found in standard laboratory manuals such as LeDoux (Ed.) (2005), Animal Models of Movement Disorders (Academic Press); Chow, et al., (2008), Using Animal Models in Biomedical Research (World Scientific Publishing Co.); Weir and Blackwell (Eds.), Handbook of Experimental Immunology, Vols. I-IV (Blackwell Scientific Publications); Creighton (1993), Proteins: Structures and Molecular Properties (W.H. Freeman and Company); Sambrook and Russell (2006), Condensed Protocols from Molecular Cloning: A Laboratory Manual; and Sambrook and Russell (2002), Molecular Cloning: A Laboratory Manual (both from Cold Spring Harbor Laboratory Press); Stryer, L. (1995) Biochemistry, Fourth Ed. (W.H. Freeman); Gait (1984), "Oligonucleotide Synthesis: A Practical Approach" (IRL Press); Nelson and Cox (2000), Lehninger, Principles of Biochemistry, Third Ed. (W. H. Freeman); and Berg et al. (2002) Biochemistry, Fifth Ed. (W.H. Freeman)

Note that as used herein and in the appended claims, the singular forms "a," "an," and "the" include plural referents unless the context clearly dictates otherwise.

Where a range of values is provided, it is understood that each intervening value, between the upper and lower limit of that range and any other stated or intervening value in that stated range is encompassed within the invention. The upper and lower limits of these smaller ranges may independently be included in the smaller ranges, and are also encompassed within the invention, subject to any specifically excluded limit in the stated range. Where the stated range includes one or both of the limits, ranges excluding either both of those included limits are also included in the invention.

In the following description, numerous specific details are set forth to provide a more thorough understanding of the present invention. However, it will be apparent to one of skill in the art that the present invention may be practiced without one or more of these specific details. In other instances, well-known features and procedures well known to those skilled in the art have not been described in order to avoid obscuring the invention.

### Methods

Described herein are methods for treating diseases and conditions associated with inflammation by administering to a subject a vector expressing an interleukin-10 (IL-10) coding sequence and an interleukin-10 receptor type 1 (IL-10R1) coding sequence. In preferred embodiments, the IL-10 and IL-10R1 peptides are expressed from a single expression vector; however, in alternative embodiments, the IL-10 and IL-10R1 coding sequences are expressed from different expression vectors. In some embodiments, the IL-10/IL-10R1 expression vector(s) is administered to a subject via, e.g., intrathecal administration (for treating, e.g., chronic pain, neuroflammation, MS or autoimmune diseases) or by intra-articular injection (for treating joint inflammation). In yet other embodiments, the IL-10/IL-10R1 expression vector is administered to a subject via cell therapy; that is, the IL-10/IL-10R1 expression vector is first used to transform or transduce antigen-presenting cells-including antigen-presenting cells taken from a subject-then the antigen-presenting cells are administered to a subject. The methods may be used to treat inflammation and physiological damage caused by inflammation associated with any disease or condition including but not limited to chronic pain, MS, autoimmune diseases, neuroinflammation, and joint inflammation.

It has been found that significant suppression of a large spectrum of inflammatory mediators can be achieved through the actions of the anti-inflammatory cytokine interleukin 10 (IL-10). IL-10 is a natural product of both astrocytes and microglia, and binds to receptors expressed by these cells producing autocrine regulation of localized inflammatory responses produced by these cells. Because of the complexity of inflammation, any agent with the capacity to re-establish normal microglial function must be able to target many different subsystems simultaneously. IL-10 forms a tripartite complex with two receptors: IL-10R1 and IL-10R2; binding primarily to IL-10R1 (Ding et al., J. Immunol., 167(12):6884-92 (2001)) where this complex then engages IL-10R2. IL-10R2 is a more abundant and promiscuous signaling subunit than IL-10R1 that also complexes with IL-22 and its primary receptor (Kotenko et al., J. Biol. Chem., 276(4):2725-32 (2001)). IL-10R1 is present at low levels in the cell membrane of antigen-presenting cells and is upregulated by IL-10 signaling and by inflammatory mediators like LPS (Ledeboer et al., Eur. J. Neurosci., 16(7): 1175-85 (2002)). The upregulation enhances signaling through the IL-10R2 receptor activating downstream effector molecules, such as JAK1, TYK2 and STAT 1 and STAT 3, and enhances signaling indirectly through PI3K-AKT. This coordinated signaling is responsible for the anti-inflammatory effect of IL-10 as well as its ability to stimulate IgG production and B-cell proliferation, even as it down-regulates antigen presentation on antigen-presenting cells, such as macrophages and T-cells. When IL-10 over-stimulates target cells, two members of a class of eight proteins called Suppressor of Cytokine Signaling (SOCS) are induced (Ding et al., J. Immunol., 170(3):1383-91 (2003), Kazi et al., Cell Mol. Life Sci., 71(17):3297-3310 (2014)). SOCS1 and SOCS3 are induced by IL-10 in a concentration-dependent manner, but SOCS1 specifically inhibits IL-10 signaling (Ding et al. 2003, *supra*) and IL-10R1 is ubiquitinated by SOCS3, an E3 ubiquitin ligase, thereby down-regulating membrane-bound IL-10R1 by proteasomal targeting (Wei et al., J. Interferon Cytokine Res., 26(5):281-90 (2006)). The present inventors have found that, at concentrations that exceed about 0.5 ng/mL, IL-10 paradoxically inhibits its own activity. Without being bound by any one theory, this inhibition may explain why virally-mediated IL-10 expression, although constitutive, has an early therapeutic effect on neuropathic pain that wears off relatively quickly, whereas intrathecal plasmid IL-10 provides up to 12 weeks of effect. That is, it is this phenomenon that explains why very high levels of adenovirus-driven cerebrospinal fluid IL-10 concentrations (~10 ng/mL) result in a lack of therapeutic effect on pain in contrast to the greater effect on pain from much lower concentrations of cerebrospinal fluid IL-10 (∼150 pg/mL) seen after IL-10 plasmid injection. The present invention thus provides a second generation therapy that dramatically widens the therapeutic window of IL-10 by coexpressing IL-10 with its primary receptor IL-10R1 to achieve constitutive autocrine signaling on antigen-presenting (IL-10R2-positive) cells.

Described herein are methods and therapeutic anti-inflammatory compositions for treating inflammatory diseases and conditions, as well as the symptoms and physiological damage associated with inflammatory diseases. Also disclosed is the use of the methods and therapeutic anti-inflammatory compositions of the disclosure in research of inflammatory diseases, including identifying pharmaceuticals, small molecules and/or biologics that may be used in conjunction in a "cocktail" with the therapeutic compositions of the present invention. The methods comprise the step of administering to a subject an IL-10/IL-10R1 expression vector comprising an IL-10 coding sequence and an IL-10R1 coding sequence. The IL-10/IL-10R1 expression vectors described herein are generally suspended in a diluent to form a therapeutic composition. The anti-inflammatory therapeutic compositions may consist of a single "naked" bacterial vector or viral vector capable of expressing both of the IL-10 and IL-10R1 coding sequences and transforming or transducing antigen-presenting cells in a subject, two bacterial or viral vectors where one vector encodes the IL-10 peptide and the vector encodes the IL-10R1 peptide, encapsulated vectors, or transduced antigen-presenting cells expressing IL-10 and IL-10R1.

### Vectors and vector components

The IL-10/IL-10R1 expression vector used in some embodiments of the methods described herein comprises a bacterial backbone (plasmid DNA) or a viral backbone; an IL-10 coding sequence; an IL-10R1 coding sequence; optionally, at least one nuclear targeting sequence 5' (upstream), 3' (downstream) or both of the IL-10 coding sequence and/or the IL-10R1 coding sequence; an internal ribosome entry site (IRES) or self-cleaving peptide; and at least one promoter and one or more other DNA control sequences. Though the embodiment where the IL-10 and IL-10R1 peptides are expressed from a single vector is preferred and is the embodiment detailed in the instant specification, it is to be understood that the IL-10 peptide and the IL-10R1 peptide can be encoded on separate vectors and codelivered directly to a subject, or to a recipient antigen-presenting cell that is then delivered to a subject. Optionally, the IL-10/IL-10R1 expression vector also comprises one or more marker sequences to allow for selection of transformed cells during preparation of the IL-10/IL-10R1 expression vector or after delivery into recipient antigen-presenting cells.

The IL-10/IL-10R1 expression vector comprises at least one IL-10 coding sequence. IL-10 may be used in wild-type form, or the IL-10 may be a mutant IL-10. One mutant IL-10 of particular interest contains one or more mutations that cause amino acid substitutions, additions or deletions as compared to wildtype IL-10 in the "hinge" region of the IL-10 protein. The human IL-10 protein is a homodimer, where each monomer comprises six alpha helices A→F, the length of which are 21, 8, 19, 20, 12 and 23 amino acids, respectively. Helices A→D of one monomer non-covalently interact with helices E and F of a second monomer, forming a non-covalent V-shaped homodimer. The "hinge" region targeted for mutation according to the present invention comprises the amino acids between the D and E alpha helices on one or both monomers of wildtype IL-10. For example, mutant rat and human IL-10 proteins have been described in which the phenylalanine at position 129 of the wildtype sequence has been replaced with a serine residue. (See, *e.g*., Sommer, et al., WO2006/130580 and Milligan, et al., Pain, 126:294-308 (2006).) The resulting mutant IL-10 is referred to as IL-10^{F129S}. Other substitutions for the wildtype phenylalanine at amino acid position 129 may be, *e.g.,* threonine, alanine, or cysteine. Thus the present invention in yet another aspect encompasses one or more substitutions at amino acid position 129 or at other amino acids within the hinge region of the IL-10 protein.

IL-10R1 (NCBI Reference Sequence for human: NP_001549.2) is a glycoprotein with a single transmembrane domain expressed on the surface of certain cell types, chiefly antigen-presenting cells. IL-10R1 binds IL-10 and is essential for the biological activity of IL-10. The binding of IL-10 to its cell surface receptors activates the JAK-STAT signal transduction pathway. Following the ligand-receptor interaction, Jak1 (associated with IL-10R1) and Tyk2 (associated with IL-10R2), members of the receptor-associated Janus tyrosine kinases (JAK) family, are phosphorylated. IL-10R1 is subject to proteasomal degradation after ubiquitination by SOCS3.

In some embodiments, the vector can be a bacterial, phage or cosmid vector. A bacterial vector can utilize any bacterial backbone known to those with skill in the art. Backbones typically selected are those that, e.g., contain or lack appropriate restriction sites to allow ease of cloning, may be produced and isolated with ease, are not immunogenic, and the like. Exemplary vectors that may be used include but are not limited to those derived from recombinant bacteriophage DNA, plasmid DNA or cosmid DNA. For example, plasmid vectors such as pBR322, pUC 19/18, pUC 118, 119 and the M13 mp series of vectors may be used. Bacteriophage vectors may include λgt10, λgt11, λgt18-23, λZAP/R and the EMBL series of bacteriophage vectors. Cosmid vectors that may be utilized include, but are not limited to, pJB8, pCV 103, pCV 107, pCV 108, pTM, pMCS, pNNL, pHSG274, COS202, COS203, pWE15, pWE16 and the charomid 9 series of vectors. Additional vectors include bacterial artificial chromosomes (BACs) based on a functional fertility plasmid (F-plasmid), yeast artificial chromosomes (YACs), and DNA constructs derived from the DNA of P1 bacteriophage (PACS).

In alternative embodiments the vector is a viral vector. In general, the five most commonly used classes of viral systems used in gene therapy can be categorized into two groups according to whether their genomes integrate into host cellular chromatin (oncoretroviruses and lentiviruses) or persist in the cell nucleus predominantly as extrachromosomal episomes (adeno-associated virus, adenoviruses and herpesviruses).

One viral delivery system useful with the IL-10 expression constructs of the present invention is a system based on viruses from the family Retroviridae. Retroviruses comprise single-stranded RNA animal viruses that are characterized by two unique features. First, the genome of a retrovirus is diploid, consisting of two copies of the RNA. Second, this RNA is transcribed by the virion-associated enzyme reverse transcriptase into double-stranded DNA. This double-stranded DNA or provirus can then integrate into the host genome and-be passed from parent cell to progeny cells as a stably-integrated component of the host genome.

In some embodiments, lentiviruses are the preferred members of the retrovirus family for use in the present invention particularly in embodiments where transduced antigen-presenting cells are the therapeutic anti-inflammatory composition that is administered to a subject. Lentivirus vectors are often pseudotyped with vesicular stomatitis virus glycoprotein (VSV-G), and have been derived from the human immunodeficiency virus (HIV), the etiologic agent of the human acquired immunodeficiency syndrome (AIDS); Visna Maedi virus, which causes encephalitis (Visna) or pneumonia in sheep; equine infectious anemia virus (EIAV), which causes autoimmune hemolytic anemia and encephalopathy in horses; feline immunodeficiency virus (FIV), which causes immune deficiency in cats; bovine immunodeficiency virus (BIV) which causes lymphadenopathy and lymphocytosis in cattle; and simian immunodeficiency virus (SIV), which causes immune deficiency and encephalopathy in non-human primates. Vectors that are based on HIV generally retain <5% of the parental genome, and <25% of the genome is incorporated into packaging constructs, which minimizes the possibility of the generation of reverting replication-competent HIV. Biosafety has been further increased by the development of self-inactivating vectors that contain deletions of the regulatory elements in the downstream long-terminal-repeat sequence, eliminating transcription of the packaging signal that is required for vector mobilization. The main advantage to the use of lentiviral vectors is that gene transfer is persistent in most tissues or cell types.

In another embodiment of the present invention, viruses from the Parvoviridae family are utilized. The Parvoviridae comprises a family of small single-stranded, non-enveloped DNA viruses with genomes approximately 5000 nucleotides long. Included among the family members is adeno-associated virus (AAV), a dependent parvovirus that by definition requires co-infection with another virus (typically an adenovirus or herpesvirus) to initiate and sustain a productive infectious cycle. In the absence of such a helper virus, AAV is still competent to infect or transduce a target cell by receptor-mediated binding and internalization, penetrating the nucleus in both non-dividing and dividing cells.

Adenoviruses (Ads) are a relatively well characterized homogenous group of viruses, including over 50 serotypes. See, e.g., International PCT Application No. WO 95/27071. Adenoviruses are medium-sized (90-100 nm), nonenveloped (without an outer lipid bilayer) icosahedral viruses composed of a nucleocapsid and a double-stranded linear DNA genome. There are 57 described serotypes in humans, which are responsible for 5-10% of upper respiratory infections in children, and many infections in adults as well. They are classified as group I under the Baltimore classification scheme, meaning their genomes consist of double-stranded DNA, and are the largest non-enveloped viruses. Because of their large size, they are able to be transported through the endosome (i.e., envelope fusion is not necessary). The virion also has a unique "spike" or fiber associated with each penton base of the capsid that aids in attachment to the host cell via the coxsackie-adenovirus receptor on the surface of the host cell. The adenovirus genome is linear, non-segmented double-stranded (ds) DNA that is between 26 and 45 kb, allowing the virus to theoretically carry 22 to 40 genes. Although this is significantly larger than other viruses in its Baltimore group, it is still a very simple virus and is heavily reliant on the host cell for survival and replication. Once the virus has gained entry into the host cell, the endosome acidifies, which alters virus topology by causing capsid components to disassociate. With the help of cellular microtubules, the virus is transported to the nuclear pore complex, where the adenovirus particle disassembles. Viral DNA is subsequently released, which can enter the nucleus via the nuclear pore. After this the DNA associates with histone molecules. Thus, viral gene expression can occur and new virus particles can be generated.

Unlike lentiviruses, adenoviral DNA does not integrate into the genome and is not replicated during cell division. Recombinant adenovirus-derived vectors, particularly those that reduce the potential for recombination and generation of wild-type virus, have also been constructed. See, International PCT Application Nos. WO 95/00655 and WO 95/11984.

Other viral or non-viral systems known to those skilled in the art also may be used to deliver IL-10/IL-10R1 expression vectors of the present invention to a subject, including but not limited to gene-deleted adenovirus-transposon vectors that stably maintain virus-encoded transgenes in vivo through integration into host cells (see Yant, et al., Nature Biotech. 20:999-1004 (2002)); systems derived from Sindbis virus or Semliki forest virus (see Perri, et al., J. Virol. 74(20):9802-07 (2002)); systems derived from Newcastle disease virus or Sendai virus; or mini-circle DNA vectors devoid of bacterial DNA sequences (see Chen, et al., Molecular Therapy. 8(3):495-500 (2003)). Mini-circle DNA as described in U.S. Patent Publication No. 2004/0214329 discloses vectors that provide for persistently high levels of nucleic acid transcription.

In addition to the IL-10 and IL-10R1 coding sequences, nuclear targeting sequences may be present in the IL-10/IL-10R1 expression vectors of the present invention. Nuclear targeting sequences are sequences that promote expression of the proteins encoded by the IL-10 coding sequence and the IL-10R1 coding sequences. For example, in one aspect the nuclear targeting sequences may bind to nuclear transport chaperone proteins, facilitating uptake of the plasmid DNA by the cell nucleus. Such sequences include but are not limited to interspersed (or dispersed) DNA repeats or repetitive sequences such as transposable elements, flanking or terminal repeats such as the long terminal repeats (LTRs) on retrovirus genomes such as SV40s, tandem repeats, and the inverted terminal repeats (ITRs) of viral genomes such as Adeno-Associated Virus and Adenovirus. In other aspects, the nuclear targeting sequences are sequences that act to bind transcription factors for import into the nucleus, such as enhancer sequences.

In addition to a vector backbone, IL-10 and IL-10R1 coding sequences and, optionally, one or more nuclear targeting sequences, the IL-10/IL-10R1 expression vector of the present invention comprises one or more DNA control sequences, such as promoter sequences, polyadenylation signals, transcription termination sequences, upstream regulatory domains, origins of replication, internal ribosome entry sites and the like, which collectively provide for the replication, transcription and translation of the IL-10/IL-10R1 coding sequences in a recipient cell. Not all of these control sequences need always be present so long as IL-10/IL-10R1 coding sequences are capable of being replicated, transcribed and translated in an appropriate host cell.

In particular, the IL-10/IL-10R1 expression vectors of the present invention comprise at least one promoter driving transcription of the IL-10 and IL10R1 coding sequences. In preferred embodiments, this promoter is a constitutive promoter. The term "constitutive" when made in reference to a promoter means that the promoter directs transcription of an operably linked nucleic acid sequence in the absence of a specific stimulus (e.g., heat shock, chemicals, light, etc.). Typically, constitutive promoters are capable of directing expression of a coding sequence in substantially any cell and any tissue. The promoters used to transcribe the IL-10/I1-10R1 peptides preferably are constitutive promoters, such as the promoters for ubiquitin, CMV, β-actin, histone H4, EF-1α or PGK genes controlled by RNA polymerase II, or promoter elements controlled by RNA polymerase I. In preferred embodiments, promoter elements controlled by RNA polymerase III are used, such as the U6 promoters (U6-1, U6-8, U6-9, e.g), H1 promoter, 7SL promoter, the human Y promoters (hY1, hY3, hY4 and hY5), the human MRP-7-2 promoter, Adenovirus VA1 promoter, human tRNA promoters, the 5s ribosomal RNA promoters, as well as functional hybrids and combinations of any of these promoters.

In alternative cases, the IL-10/I1-10R1 coding sequences may be under the control of an inducible promoter, such as tetracycline-controlled transcriptional activation where transcription is reversibly turned on (Tet-On) or off (Tet-Off) in the presence of the antibiotic tetracycline or a derivative thereof, such as doxycycline. In a Tet-Off system, expression of tetracycline response element-controlled genes can be repressed by tetracycline and its derivatives. Tetracycline binds the tetracycline transactivator protein, rendering it incapable of binding to the tetracycline response element sequences, preventing transactivation of tetracycline response element-controlled genes. In a Tet-On system on the other hand, the tetracycline transactivator protein is capable of initiating expression only if bound by tetracycline; thus, introduction of tetracycline or doxycycline initiates the transcription of IL-10/I1-10R1 peptides. Another inducible promoter system known in the art is the estrogen receptor conditional gene expression system. Compared to the Tet system, the estrogen receptor system is not as tightly controlled; however, because the Tet system depends on transcription and subsequent translation of a target gene, the Tet system is not as fast-acting as the estrogen receptor system.

The methods of the disclosure are drawn to co-expression of the IL-10 and IL-10R1 peptides in the same antigen-presenting cell. To achieve this, one of ordinary skill in the art may employ a number of techniques including co-transfection of two or more plasmids, the use of multiple or bidirectional promoters, or, preferably, the creation of bicistronic or multicistronic vectors. Unlike promoters that create unique mRNA transcripts for each gene that is expressed, multicistronic vectors simultaneously express two or more separate peptides-in this case the IL-10 and IL-10R1 peptides-from the same mRNA. Translation in eukaryotes usually begins at the 5' cap so that only a single translation event occurs for each mRNA. However, some bicistronic vectors take advantage of an element called an Internal Ribosome Entry Site (IRES) to allow for initiation of translation from an internal region of the mRNA.

Alternatively, "self-cleaving" 2A peptides may be used in lieu of IRES elements in muliticistronic vectors. Self-cleaving peptides are short (about 20 amino acids) and produce equimolar levels of multiple genes from the same, single, mRNA. The "cleavage" occurs between the glycine and proline residues found on the C-terminus of the self-cleaving peptide which is positioned between the coding regions of the two different peptides. Common 2A self-cleaving peptides include peptides T2A, P2A, E2A and F2A.

Optionally, the vector of the present invention also comprises a selection marker gene, such as that coding for antibiotic resistance. Marker genes of use in the present invention include but are not limited to human nerve growth factor receptor (detected with a monoclonal antibody (MAb), such as described in U.S. Pat. No. 6,365,373); truncated human growth factor receptor (detected with a MAb); mutant human dihydrofolate reductase (DHFR; fluorescent MTX substrate available); secreted alkaline phosphatase (SEAP; fluorescent substrate available); human thymidylate synthase (TS; confers resistance to anticancer agent fluorodeoxyuridine); human glutathione S-transferase alpha (GSTA1; conjugates glutathione to the stem cell selective alkylator busulfan; chemoprotective selectable marker in CD34+ cells); CD24 cell surface antigen in hematopoietic stem cells; human CAD gene to confer resistance to N-phosphonacetyl-L-aspartate (PALA); human multi-drug resistance-1 (MDR-1; P-glycoprotein surface protein selectable by increased drug resistance or enriched by FACS); human CD25 (IL-2α; detectable by MAb-FITC); Methylguanine-DNA methyltransferase (MGMT; selectable by carmustine); and Cytidine deaminase (CD; selectable by Ara-C). Drug selectable markers such as puromycin, hygromycin, blasticidin, G418, tetracycline may also be employed. In addition, using FACs sorting, any fluorescent marker gene may be used for positive selection, as may chemiluminescent markers (e.g. Halotags), and the like

Where delivery of the IL-10/IL-10R1 expression vector directly to a subject via intrathecal or intra-articular injection is contemplated, the IL-10/I1-10R1 expression vector preferably is viral-based as described *supra.* However, in embodiments where transformed or transduced antigen-presenting cells are administered to a subject as described in more detail *infra,* it is also contemplated that antigen-presenting cells of choice can be engineered to produce human artificial chromosomes that express the IL-10 and IL-10R1 peptides. Fullyfunctional human artificial chromosomes offer several advantages over viral-based delivery systems including increased payload size, the fact that extrachromosomal maintenance avoids host-cell disruption, and transcriptional silencing of introduced genes and possible immunological complications are avoided. Currently, there are several methods for engineering human artificial chromosomes, including the "top down" method, the "bottom up" method, creating minichromosomes, and induced de novo chromosome generation. The "bottom up" approach of artificial chromosome formation relies on cell-mediated *de novo* chromosome formation following transfection of a permissive cell line with cloned α-satellite sequences, which comprise typical host cell-appropriate centromeres and selectable marker gene(s), with or without telomeric and genomic DNA. (For protocols and a detailed description of these methods see, e.g., Henning, et al., PNAS USA, 96:592-97 (1999); Grimes, et al., EMBO Rep. 2:910-14 (2001); Mejia, et al., Genomics, 79:297-304 (2002); and Grimes, et al., Mol. Ther., 5:798-805 (2002).) The "top down" approach of producing artificial chromosomes involves sequential rounds of random and/or targeted truncation of pre-existing chromosome arms to result in a pared down artificial chromosome comprising a centromere, telomeres, and DNA replication origins. (For protocols and a detailed description of these methods see, e.g., Choo, Trends Mol. Med., 7:235-37 (2001); Barnett, et al., Nuc. Ac. Res., 21:27-36 (1993); and Katoh, et al., Biochem. Biophys. Res. Commun., 321:280-90 (2004).) "Top down" artificial chromosomes are constructed optimally to be devoid of naturally-occuring expressed genes and are engineered to contain DNA sequences that permit site-specific integration of target DNA sequences onto the truncated chromosome, mediated, e.g., by site-specific DNA integrases.

A third method of producing artificial chromosomes known in the art is engineering of naturally occurring minichromosomes. This production method typically involves irradiation-induced fragmentation of a chromosome containing a functional, e.g., human neocentromere possessing centromere function yet lacking α-satellite DNA sequences and engineered to be devoid of non-essential DNA. (For protocols and a detailed description of these methods see, e.g., Auriche, et al., EMBO Rep. 2:102-07 (2001); Moralli, et al., Cytogenet. Cell Genet., 94:113-20 (2001); and Carine, et al., Somat. Cell Mol. Genet., 15:445-460 (1989).) As with other methods for generating artificial chromosomes, engineered mini-chromosomes can be engineered to contain DNA sequences that permit site-specific integration of target DNA sequences such as the IL-10 and IL-10R1 sequences. The fourth approach for production of artificial chromosomes involves induced *de novo* chromosome generation by targeted amplification of specific chromosomal segments. This approach involves large-scale amplification of peri-centromeric/ribosomal DNA regions situated on acrocentric chromosomes. The amplification is triggered by co-transfection of excess DNA specific to the pericentric region of chromosomes, such as ribosomal RNA, along with DNA sequences that allow for site-specific integration of the, e.g., IL-10 and IL-10R1 coding sequences and also a drug selectable marker which integrates into the pericentric regions of the chromosomes. (For protocols and a detailed description of these methods see, e.g., Csonka, et al., J. Cell Sci 113:3207-16 (2002); Hadlaczky, et al., Curr. Opini. Mol. Ther., 3:125-32 (2001); and Lindenbaum and Perkins, et al., Nuc. Ac. Res., 32(21):e172 (2004).) During this process, targeting to the pericentric regions of acrocentric chromosomes with co-transfected DNA induces large-scale chromosomal DNA amplification, duplication/activation of centromere sequences, and subsequent breakage and resolution of dicentric chromosomes resulting in a "break-of" satellite DNA-based artificial chromosome containing multiple site-specific integration sites.

### Delivery

The IL-10/IL-10R1 expression vectors can be introduced into a subject either *in vivo* or *in vitro* (also termed *ex vivo*). *In vivo* introduction comprises administering the IL-10/IL-10R1 expression vectors directly to a subject, and *in-vitro* introduction comprises administering antigen-presenting cells that have been engineered to co-express IL-10 and IL-10R1 to a subject.

The preferred method of delivery for treating joint inflammation is joint injection (intra-articular injection), where a hypodermic needle is injected into the affected joint delivering a dose of the therapeutic anti-inflammatory composition of the present invention. In treating chronic or neuropathic pain, MS, neuroinflammation, or an autoimmune disease, intrathecal administration is preferred. Intrathecal injection involves injection of the therapeutic anti-inflammatory composition into the spinal canal or the subarachnoid space so that it reaches the cerebrospinal fluid.

Alternatively, if transduced *in vitro,* the desired antigen-presenting recipient cells are preferably removed from a subject, transformed or transduced with the IL-10/IL-10R1 expression vector and reintroduced into the subject (that is, the antigen-presenting cells are autologous). Alternatively, however, syngeneic or xenogeneic antigen-presenting cells (such as from an established antigen-presenting cell line that has been stably transformed with the IL-10/IL-10R1 expression vector) can be transformed or transduced for delivery in the subject. Antigen-presenting cells or precursors thereof that may be transformed or transduced include any cells from the monocyte family, including monoblasts, monocytes, astrocytes, oligodendrocytes, microglia, macrophages, B cells, dendritic cells foam cells, lymphoblasts, and B lymphocytes. Precursor antigen-presenting cells may be transduced and cultured in the undifferentiated state, then differentiated in vitro before delivery to the subject.

The IL-10/IL-10R1 expression vector can be delivered to the antigen-presenting cells to be engineered by any method known in the art. The terms transfection and transformation refer to the taking up of exogenous nucleic acid, e.g., an expression vector, by a host cell whether or not any coding sequences are, in fact, expressed. Numerous methods of transfection are known to the ordinarily skilled artisan, for example, by Agrobacteriummediated transformation, protoplast transformation (including polyethylene glycol (PEG)-mediated transformation, electroporation, protoplast fusion, and microcell fusion), lipid-mediated delivery, liposomes, electroporation, sonoporation, microinjection, particle bombardment and silicon carbide whisker-mediated transformation and combinations thereof; direct uptake using calcium phosphate; polyethylene glycol (PEG)-mediated DNA uptake; lipofection; microcell fusion; lipid-mediated carrier systems; or other suitable methods. Successful transfection is generally recognized by detection of the presence of IL-10/IL-10R1 gene transcripts or IL-10/IL-10R1peptides within the transfected cell.

Because viral vectors are a preferred embodiment of the invention, antigen-presenting cells are preferably transduced using the viral vector. The use of viral infection is unique in that a virus' naturally occurring means of introducing its genetic material into a cell is taken advantage of to transfer a nucleic acid molecule of interest into a cell. As discussed *supra,* examples of viruses modified and applied to such techniques include adenoviruses, adeno-associated viruses, and retroviruses. Generally, nucleic acid molecules of interest may be cloned into a viral genome. Upon replication and packaging of the viral genome, the resultant viral particle is capable of delivering the nucleic acid of interest into a cell via the viral entry mechanism. Commonly, the viral genome is first made replication deficient by nucleic acid manipulation before the addition of the nucleic acid of interest. The resultant viral genome, or viral vector, requires the use of a helper virus or a packaging system to complete viral particle assembly and release from a cell.

If the IL-10/IL-10R1 expression vectors are to be administered to a subject directly, via, e.g., intrathecal or intra-articular injection, the expression vectors may optionally be encapsulated for delivery. Techniques for encapsulating the IL-10/IL-10R1expression vector vary depending on the type of microparticles used and such techniques are described in, e.g., Chavez, et al., USSN. 14/905,915. The microparticles may be comprised of any biodegradable polymer. To be used successfully as a biodegradable polymer in the controlled drug delivery formulations of the present invention, the material must be chemically inert and free of leachable impurities. Ideally the polymer also has an appropriate physical structure, with minimal undesired aging, and is readily processable. Some of the materials include poly(2-hydroxy ethyl methacrylate), poly(N-vinyl pyrrolidone), poly(methyl methacrylate), poly(vinyl alcohol), poly(acrylic acid), polyacrylamide, poly(ethylene-co-vinyl acetate), poly(ethylene glycol), and poly (methacrylic acid). Biodegradable polymers of particular use in the present invention include polylactides (PLA), polyglycolides (PGA), poly(lactide-co-glycolides) (PLGA), polyanhydrides, polycaprolactone, poly-3-hydroxybutyrate and polyorthoesters. Such biodegradable polymers have been characterized extensively and can be formulated to exhibit desired degradation properties as is known in the art (see, *e.g.,* Edlund & Albertsson, Degradable Aliphatic Polyesters, pp. 67-112 (2002), Barman, et al., J. of Controlled Release, 69:337-344 (2000); Cohen, et al., Pharmaceutical Res., (8): 713-720 (1991)).

In one particular case, the polymer comprises poly(lactideco-glycolides) (PLGA). PLGA is a copolymer which is used in a host of FDA approved therapeutic devices, owing to its biodegradability and biocompatibility. Depending on the ratio of lactide to glycolide used for the polymerization, different forms of PLGA can be obtained: these are usually identified in regard to the monomers' ratio used (*e.g.,* PLGA 75:25 identifies a copolymer whose composition is 75% (molar percent) lactic acid and 25% (molar percent) glycolic acid). PLGA degrades by hydrolysis of its ester linkages in the presence of water. It has been shown that the time required for degradation of PLGA is related to the monomers' ratio used in production: the higher the content of glycolide units, the lower the time required for degradation. An exception to this rule is the copolymer with 50:50 monomers' ratio which exhibits the faster degradation (about two months). In addition, polymers that are end-capped with esters (as opposed to the free carboxylic acid) demonstrate longer degradation half-lives. Alternatively, the IL-10/IL-10R1 expression vector may be encapsulated in batches of microparticles having different release profiles; for example, 10% of the expression vector to be delivered may be encapsulated in microparticles having, *e.g.,* a one day to four week release profile; 30% of the expression vector to be delivered may be encapsulated in microparticles having, *e.g.,* a three week to six week release profile; 30% of the expression vector to be delivered may be encapsulated in microparticles having, *e.g.,* a six week to ten week release profile; and 30% of the expression vector to be delivered may be encapsulated in microparticles having, *e.g.,* an eight week to twelve week release profile. In such a case, a single type of biodegradable polymer may be used, but used in formulations with different release profiles; alternatively, different biodegradable polymers having different release characteristics may be used.

Once microparticles are obtained, they are suspended in an acceptable diluent to form a therapeutic composition for administration to an animal. Such diluents (or excipients) include any pharmaceutical agent that does not itself induce the production of antibodies harmful to the individual receiving the composition, and that may be administered without undue toxicity. Pharmaceutically acceptable diluents may comprise sorbitol, alum, dextran, sulfate, large polymeric anions, any of the various TWEEN compounds, and liquids such as water, saline, glycerol or ethanol, oil and water emulsions, or adjuvants such as Freund's adjuvant.

Because the methods and therapeutic anti-inflammatory compositions of the disclosure do not significantly induce an immune response or dose tolerance in subjects, the can be used and/or administered as needed for therapeutic effect. That is, the therapeutic anti-inflammatory composition can be delivered approximately every 30-90 days (or as required according to, e.g., the vector type (bacterial, viral (integrative or not), artificial chromosome), and the degradation profile of the biodegradable polymer) as needed for therapeutic effect for shorter-term therapy. For example, when longer-term therapy is desired, the therapeutic composition can be delivered approximately every 90 days as needed for therapeutic effect for greater than one year; and if necessary, for the life of the subject.

Dosage ranges of the therapeutic anti-inflammatory compositions used in the methods of the present invention vary from subject to subject, depending on the species, age, and general condition of the subject, the severity of the condition being treated and the particular IL-10/IL-10R1 expression vector to be delivered, mode of administration, and the like. For example, dosage ranges include a therapeutically effective dose at 10-1000 µg vector DNA per kg, 20-500 µg vector DNA per kg, 25-250 µg vector DNA per kg, or 50-100 µg vector DNA per kg depending on the desired duration and anatomical location of the injection.

The IL-10/IL-10R1 expression vectors or microparticles containing the IL-10/IL-10R1 expression vectors used in the methods of the present invention may be co-administered in a "cocktail" with other therapeutic agents useful in treating inflammation including but not limited to glucocorticoids; methotrexate; hydroxychlolquine; sulfasalazine; lefunomide; anti-TNF agents such as etanercept, infliximab and adalimumab; abatacept; nonsteroidal anti-inflammatory drugs (NSAIDs); Glatiramer acetate and interferon B, Mitoxantrone, and Natalizumab. Additionally, the IL-10/IL-10R1 expression vectors used in the methods of the present invention may be co-administered with cells, such as stem cells bioengineered to express IL-10/IL-10R1 expression vector. Generally, any method known in the art can be used to monitor success of treatment in humans, including both clinical and phenotypic indicators.

### Conditions to be treated

Because the compositions and therapeutic anti-inflammatory compositions of the present invention overcome the down-regulation of IL-10 to achieve robust IL-10-mediated suppression of inflammation, they thus are efficacious in treating a number of diseases and conditions. For example, the methods and therapeutic anti-inflammatory compositions of the present invention can be used to treat symptoms and damages caused by multiple sclerosis (MS). MS is a chronic, often debilitating autoimmune disease of the central nervous system. The disease afflicts approximately 2.5-3 million people worldwide, and ~400K people in the US, with 200 people per week being diagnosed. At onset, -85% of patients present with a relapsing remitting disease (RRMS), 10% with primary progressive disease (PPMS) and 5% with progressive relapsing disease (PRMS). Full neurological recovery usually occurs after the first episode of RRMS, but ~50% of those patients over a 10-year period will accumulate increasingly persistent neurological deficits at subsequent relapses and convert to a secondary progressive phase of the disease which becomes increasingly disabling. The clinical symptoms of MS are highly variable from patient to patient. The list of symptoms includes impairment of cognition, function, loss of vision, weakness, spasticity, lack of coordination, imbalance, fatigue, sexual, bowel and bladder dysfunction, paresthesias and pain. Clinically significant pain is experienced by as many as 65% of patients over the course of their disease and represents one of the most disabling, though under recognized and frequently inadequately treated symptoms. The pain is usually neuropathic, and can vary in nature dependent on the damaged neurons involved. It should also be noted that pain is a prominent MS symptom that is poorly treated by current therapies.

In addition to MS, other conditions mediated by a loss of myelin can be treated using the methods and therapeutic anti-inflammatory compositions of the present invention. These conditions include neuroinflammation (i.e., neuroinflammation caused by trisomy 21), ischemic demyelination conditions, inflammatory demyelination conditions, pediatric leukodystrophies, mucopolysaccharidosis, perinatal germinal matrix hemorrhage, cerebral palsy, periventricular leukoinalacia, radiation-induced conditions, and subcortical leukoencephalopathy due to various etiologies, as well as mental illnesses such as schizophrenia. Ischemic demyelination conditions include cortical stroke, lacunar infarct, post-hypoxic leukoencephalopathy, diabetic leukoencephalopathy, and hypertensive leukoencephalopathy. Inflammatory demyelination conditions include multiple sclerosis, Schilder's Disease, transverse myelitis, optic neuritis, post-vaccination encephalomyelitis, and post-infectious encephalomyelitis. Pediatric leukodystrophy conditions include lysosomal storage diseases (e.g., Tay-Sachs Disease), Canavan's Disease, Pelizaeus-Merzbacher Disease, and Crabbe's Globoid body leukodystrophy. An example of mucopolysaccharidosis is Sly's Disease. Radiation-induced conditions include radiation-induced leukoencephalopathy and radiation induced myelitis. Etiologies causing subcortical leukoencephalopathy include HIV/AIDS, head trauma, and multi-infarct states.

Additionally, the methods and therapeutic anti-inflammatory compositions of the present invention also may be used to treat joint inflammation is in the knee, elbow, wrist, ankle, hip, shoulder, or spine. Conditions treatable by the methods and therapeutic anti-inflammatory compositions of the present invention include rheumatoid arthritis and osteoarthritis, as well as tendonitis, bursitis, inflammation of the ligament, synovitis, gout, and systemic lupus erythematosus.

Yet another use of the methods and therapeutic anti-inflammatory compositions of the present invention is the treatment of autoimmune diseases. Autoimmune diseases represent an attack by the host immune system on specific targets in host tissues leading to critical loss of function and pathological symptoms. The classic example of this phenomenon is the presence in patients with *Myasthenia Gravis* of antibodies directed at the nicotinic acetylcholine receptor present at the neuromuscular junction. Because this receptor is so important in transmitting signals from motor neurons to muscles, such antibodies cause partial paralysis and other neuromuscular deficits. It is unclear why such auto-antibodies arise but the only treatment with any efficacy has been plasmapheresis; that is, replacement of the plasma fraction of blood to reduce the circulating concentration of the pathologic antibody. Although in many cases, dominant antibodies have been identified that explain the specific disease pathology, there has been no real progress in treating such diseases. This may be in part because investigators persist in focusing on the pathogenic antibodies and their effects rather than thinking of autoimmune diseases as a failure of tolerance.

Immune tolerance is defined as a curtailed response to administration of an antigen in animals previously seropositive for antibodies against that antigen. The adaptive immune system can be taught to ignore a given antigen by exposing the animal to escalating doses of antigen. This is called desensitization and it is used primarily in humans to treat allergies of various kinds, e.g. peanut allergy. However, this is a slow and uncertain process, frequently ineffective. How reactive the immune system is to a particular antigen is driven in part by the balance between reactive, cytotoxic B- & T-lymphocytes and more recently discovered regulatory B- & T-cells. Stimulation of these cells with IL-10 induces a switch from cytotoxic to tolerant phenotype. In fact this switch results in secretion of IL-10 from these same cells, called regulatory B- and T-cells. Thus exposure of T- and B-cells to exogenous IL-10 is predicted to induce tolerance to antigens. In practice, however, getting the concentration of exogenous IL-10 at the right concentration to effect this transition is difficult. Combined IL-10 and IL-10R1 expression in these cells is predicted to induce phenotype switching that is somewhat independent of IL-10 concentration.

Autoimmune diseases that can be treated or ameliorated with the methods and therapeutic anti-inflammatory compositions of the present invention include but are not limited to myocarditis, lupus nephritis and other lupus disorders, interstitial systitis, autoimmune hepatitis, alopecia areata, epidermolysis bullosa acquisita, Addison's disease, Diabetes mellitus type 1, Grave's disease, Celiac disease, Crohn's disease, ulcerative colitis, aplastic anemia and other anemias, ankylosing spondylitis, Felty syndrome, psoriatic arthritis, systemic lupus, Schnitzler syndrome, fibromyalgia, myasthenia gravis, Guillain-Barre syndrome, Lambert-Eason myasthenic syndrome, autoimmune retinopathy, Cogan syndrome, Grave's ophthalmopathy, scleritis, Meniere's disease, Churg-Strauss syndrome, Kawasaki's disease, rheumatoid vasculitis, and polyarteritis nodosa. In addition, conditions that are common to autoimmune diseases such as chronic fatigue syndrome, complex regional pain syndrome, eosinophilic esophagitis, gastritis, POEMS syndrome, Raynaud's phenomenon, primary immunodeficiency, and pyoderma gangrenosum may be treated with the methods and therapeutic anti-inflammatory compositions of the present invention.

Additional objects, advantages, and novel features of this invention will become apparent to those skilled in the art upon examination of the following examples thereof, which are not intended to be limiting. In the Examples, procedures that are constructively reduced to practice are described in the present tense, and procedures that have been carried out in the laboratory are set forth in the past tense.

### EXAMPLES

The following examples are put forth so as to provide those of ordinary skill in the art with a complete disclosure and description of how to make and use the present invention, and are not intended to limit the scope of what the inventors regard as their invention, nor are they intended to represent or imply that the experiments below are all of or the only experiments performed. It will be appreciated by persons skilled in the art that numerous variations and/or modifications may be made to the invention as shown in the specific embodiments without departing from the scope of the invention as broadly described. The present embodiments are, therefore, to be considered in all respects as illustrative and not restrictive.

Efforts have been made to ensure accuracy with respect to numbers used (*e.g.,* amounts, temperature, etc.) but some experimental errors and deviations should be accounted for. Unless indicated otherwise, parts are parts by weight, molecular weight is weight average molecular weight, temperature is in degrees centigrade, and pressure is at or near atmospheric.

### Example 1-Expression Vectors

XT-250 is an expression plasmid that contains the rat IL-10 cDNA, used in these studies simply because rIL-10 engages with the rat IL-10R1 receptor considerably better than human IL-10 (hIL-10). XT-250, like its hIL-10 counterpart-XT-150-is a solution of DNA in D-mannose. Regardless of species, all of the IL-10 cDNAs used contain a point mutation yielding an amino acid change, F129S, that has been found to yield significantly longer duration of efficacy in pain models (Milligan et al., Pain, 126(1-3):294-308 (2006)). The plasmid backbone contains a Kanamycin resistance gene, CMV promoter, β-globin intron, growth hormone polyA and 2 AAV2 ITRs. This plasmid was also used to make AAV9 vectors.

LV02 is an expression plasmid that contains the above control elements with a cDNA downstream of the CMV promoter comprised of the hIL-10R1 coding sequence followed by a 2a, self-cleaving peptide, and the hIL-10 coding sequence. Test transfection of HT-1080 cells revealed surface expression of IL-10R1 and secretion of IL-10 into the medium (2.1 ng/mL).

### Example 2-Supporting Data

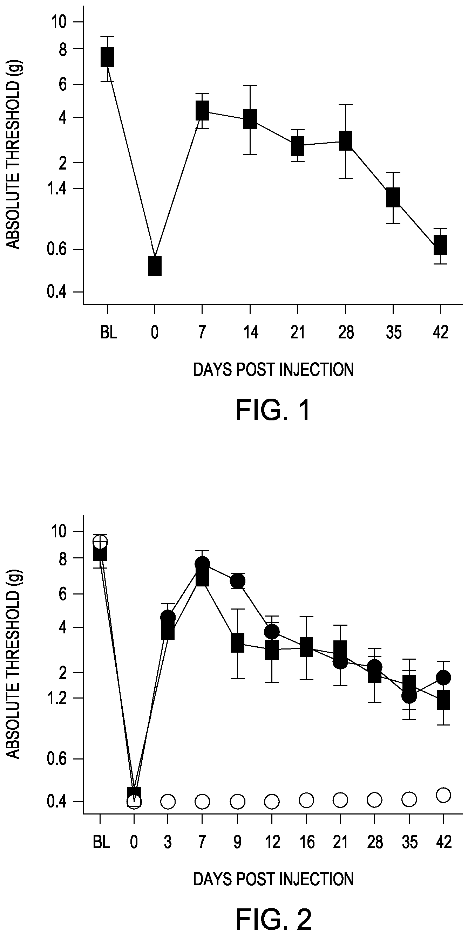

It should be noted that IL-10 displays some idiosyncrasies in terms of speciesspecificity. For example, mouse IL-10 does not interact with the human IL-10 receptor, although human IL-10 does bind to the mouse IL-10 receptor, and human IL-10 binds weakly with the rat IL-10 receptor. For this reason, rat IL-10 (rIL-10) has generally been used in rat experiments, and human IL-10 (hIL-10) has been used in mouse, dog and horse studies. The fact that hIL-10 activates the rat IL-10 receptor only at high concentrations is a very useful feature, because co-expression of human IL-10R1 in target rat tissues results in full response to human IL-10. In order to simplify delivery, adeno-associated viruses (AAV) encoding rIL-10, hIL-10, or HL-10R1 were constructed. Serotype 9 was chosen because AAV9 distributes very well when injected intrathecally and transduces a wide variety of cells including antigen-presenting cells such as astrocytes that themselves express IL-10R1. As shown in Fig. 1, AAV9-hIL-10 is anti-allodynic in the rat Chronic Constriction Injury (CCI) model of neuropathic pain, but the effect wears off. However, a 1:1 mixture of AAV9-hIL-10 and AAV9-hIL10R1 directs stable elimination of allodynia in this model (Fig. 2), consistent with the hypothesis that combined IL-10 and IL-10R1 expression permits much higher intrathecal dosing to drive widespread suppression of inflammation throughout the spinal cord and white matter tracts. Open circles = AAV9-ML-10R1; closed circles = AAV9-hIL-10 + XR-101; closed squares = AAV9-hIL-10Ra + AAV9-hIL-10.

In order to simplify administration of the two cDNAs, a bicistronic vector was constructed in which the EF1α promoter directs expression of hIL-10R1 and hIL-10. The primary translation product contains a 2a self-cleaving peptide. When this plasmid was transiently transfected into HT-180 cells, hIL-10 was easily detected in the medium by ELISA (R&D Systems) at approximately 2 ng/mL. In addition, surface IL-10R1 was detected by immunofluorescence in transfected but not control cells.

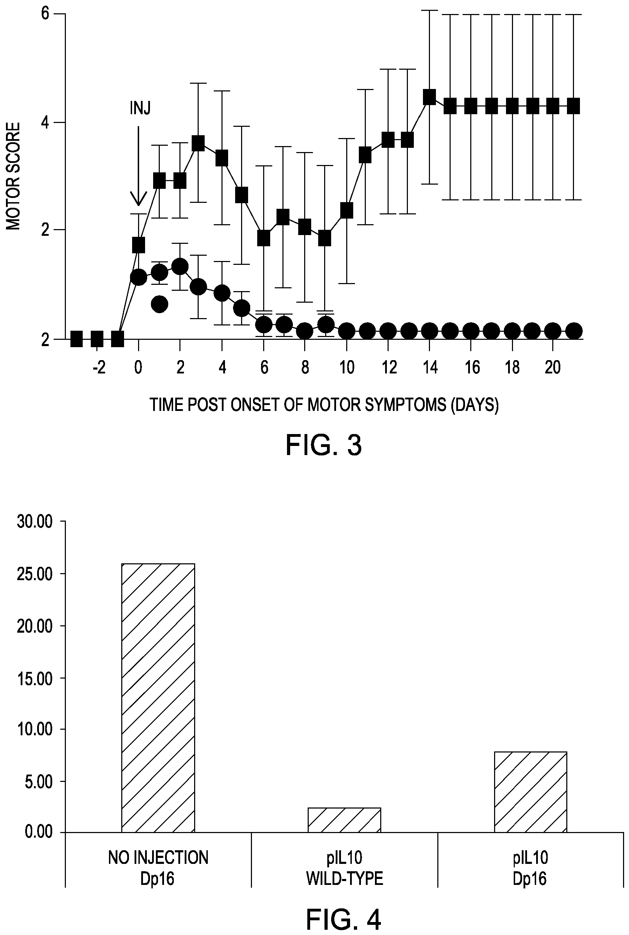

Plasmid-based gene transfer of IL-10 in relapsing-remitting EAE rats has been found to be effective (Sloane et al., Brain, Behavior and Immunity, 23(1):92-100 (2009)). In this study, plasmid encapsulated in PLGA microparticles was injected intrathecally at a time when tail paralysis had already occurred (Fig. 3). Remarkably, IL-10 reversed tail paralysis and extinguished MS-like pathology. Motor scores: 0 = normal; 1 = tail tip paralysis; 2 = full tail paralysis; 3 = hind leg weak; 4 = hind-leg paralysis; 5 = full hind-leg paralysis; 6 - partial foreleg paralysis. N = 6 per group.

### Example 3-Treatment with the IL-10/IL-10R1 expression vector to assess motor disability

Rats treated with MOG to induce a mild relapsing-remitting MS-like pathology are injected intrathecally with doses of IL-10 plasmid previously shown to be efficacious in this model (Sloane et al. 2009) at a time when symptoms such as tail paralysis are visible. In addition to IL-10 plasmid, separate cohorts of rats are dosed with equal amounts of almost identical plasmid in which the IL-10 cDNA is replaced with the IL-10/IL-10R1 cassette named LV02. The goal of this 30-day experiment is to establish whether LV02 works better than XT-250 (IL-10 expression only) at higher doses. At low plasmid doses (3 µg), little difference is expected between the two formulations. However, at high doses (∼100 µg), where loss of XT-250 efficacy is seen, continued efficacy of LV02 is expected.

Adult male Dark Agouti rats (250-300 g; Envigo), can be used to induce EAE via intradermal injection of 35 µg MOG in 0.01 M Na-Acetate (pH 3.0) emulsified in incomplete Freund's adjuvant (1:1 ratio). Approximately 1 week after injection, rats evince progressive motor impairment (and allodynia), starting with tail paralysis (motor score = 1) that leads ultimately to a score of 7 where they are euthanized. However, in the intermediate period animals spontaneously improve before relapsing. Rats (N=5 per group) are injected intrathecally (via lumbar 4/5; L4/5) with either XT-250 or LV02 in a volume of approximately 40 µl when they present with tail paralysis. Groups of 5 rats are injected with PBS (control) or DNA (1, 3, 7, 10, 30 or 100 µg) either XT-250 or LV02. Animals are scored for motor disability over the next 30 days.

### Example 4-Treatment with the IL-10/IL-10R1 expression vector to assess reduction of supra-spinal lesions

A dose-ranging experiment is conducted in which doses of LV02 are achieved in which efficacy in terms of motor deficit is maintained but supra-spinal lesions are significantly reduced (H&E) and demyelination is decreased (Luxol Fast Blue). LV02, XT-250 or PBS are injected intrathecally into EAE rats (N=5 per group) at the highest doses found to be efficacious in the experiments performed in Example 3. The in-life phase of this experiment concludes when animals return to a score of 0 (normal), usually <2 weeks. Samples of CSF are taken from naive animals, the same animals at the time of LV02 administration and immediately prior to perfusion/fixation. Animals are perfused with PBS followed by PBS/4% paraformaldehyde. Spinal cords and brains are transferred to cryoprotectant (30% sucrose in PBS) prior to sectioning. Sections (40 micron) of spinal cord (longitudinal) and brain (coronal) are stained with Hematoxylin-Eosin (H&E) to identify lesions and lymphocytic infiltrates. Adjacent sections are stained with Luxol Blue to identify zones of demyelination. Sections are also stained for human IL-10R1 (Millipore # 06-1067), Iba1 (microglia), GFAP (astrocytes), and NeuN (neurons). Co-staining with MHC-II antibodies identifies activation of antigen-presenting cells. At each level of spinal cord (lumbar, thoracic, cervical), lesions identified by H&E in longitudinal sections are counted. Similarly, sections from brainstem, cerebellum, occipital cortex and frontal cortex are scored for the presence of lesions by H&E. Significant (unpaired t-test) reductions in lesions are expected in both XT-250 and LV02 in spinal cord compared to PBS controls. If LV02 works better than XT-250 in brainstem and cortical white matter tracts, a significant difference in lesions per section should be detected.

### Example 5- Treatment with the IL-10/IL-10R1 expression vector to assess biodistribution

Biodistribution of plasmid DNA throughout the brain at various doses of LV02 is evaluated. This experiment is designed to understand how plasmid distributes throughout white matter tracts and spinal cord over time. The highest feasible dose of LV02 (300 µg) is injected intrathecally via L4/5 of naive rats (N=3 per time-point). Tissues are isolated from spinal cord (lumbar, thoracic, cervical), brainstem, occipital cortex and frontal cortex 4 h, 7 and 30 days after injection. DNA is extracted from these tissues and subjected to quantitative PCR. This experiment reveal hows LV02 distributes through the brain and whether it persists over an extended period.

### Example 6-Statistical Analysis

All statistical comparisons of motor deficit score and immuno- histochemical analysis data were computed with statistical software. Motor deficit scores are analyzed via a nonparametric Wilcoxon rank sum test. The repeated measures ANOVA statistic examines group effects across time-points tested throughout experiments. Immunohistochemistry markers will be analyzed by two-tailed t-test statistic.

### Example 7: Detection of neuroinflammation in Down Syndrome model mice

In another example (Figure 4), injection of expression plasmid encoding mouse IL-10F129S into the brains of normal mice and mice engineered to mimic Down's Syndrome (dp16) showed that over-expression of IL-10 dramatically reduced expression of IL-10R1 mRNA in support of other data both from the inventors' laboratory and published findings (see, e.g.,Ding, et al., J Immunol 167(12): 6884-6892 (2001)). Figure 2 shows levels of mRNA for the signaling receptor of IL-10 (IL-10R1).. Compared to no injection controls, pDNA-IL10 treatment dramatically suppressed gene expression of IL-10R1 in both wild type and Down Syndrome mouse brains.

## Claims

1. An agent for use in treating inflammation in a subject, wherein the agent is one or more bacterial, viral, phage, cosmid, or artificial chromosome vectors expressing interleukin 10 (IL-10) and interleukin 10 type 1 receptor (IL-10R1) in antigen-presenting cells in the subject, and wherein at least one constitutive promoter drives transcription of the IL-10 and IL-10R1 coding sequences.

2. An agent for the use of claim 1, whereby the use overcomes the down-regulation of IL-10 signaling.

3. An agent for the use of claim 1 or 2, whereby the use achieves constitutive autocrine signaling on antigen-presenting (IL-10R2-positive) cells.

4. An agent for the use of any one of claims 1-3, wherein IL-10 and IL-10R1 are expressed from a single vector.

5. An agent for the use of any one of claims 1-4, wherein the vector is a viral vector, optionally wherein the viral vector is an adeno-associated virus vector or a lentivirus vector.

6. An agent for the use of any one of claims 1-5, wherein the IL-10 and IL-10R1 coding sequences are transcribed as a single mRNA.

7. An agent for the use of any one of claims 1-6, wherein the vector further comprises:
(i) a coding sequence for an internal ribosome entry site between the IL-10 and the IL-10R1 coding sequences; or
(ii) a coding sequence for a self-cleaving 2a peptide between the IL-10 and IL-10R1 coding sequences.

8. An agent for the use of any one of claims 1-7, wherein the inflammation is caused by:
(i) neuropathic or chronic pain, and the one or more vectors are delivered by intrathecal injection;
(ii) multiple sclerosis (MS), and the one or more vectors are delivered by intrathecal injection;
(iii) an autoimmune disease, and the one or more vectors are delivered by intrathecal injection; or
(iv) multiple sclerosis (MS), chronic pain, joint inflammation, neuroinflammation or an autoimmune disease.

9. An agent for the use of any one of claims 1-8, wherein the inflammation is located in a joint, optionally wherein the inflammation is neuroinflammation, and the one or more vectors are delivered by intra-articular injection.

10. An agent for the use of any one of claims 1-9, wherein the antigen-presenting cells are selected from the group consisting of monoblasts, monocytes, astrocytes, oligodendrocytes, microglia, macrophages, B cells, dendritic cells, foam cells, lymphoblasts, and B lymphocytes.

11. An agent for the use of any one of claims 1-10, wherein the antigen-presenting cells:
(i) are removed from a subject to be treated, transduced with the one or more vectors *in vitro,* and administered back to the subject; or
(ii) are stably transformed with the one or more vectors and are maintained in culture.

12. A single viral or bacterial expression vector comprising the coding sequences for interleukin 10 (IL-10) and interleukin 10 type 1 receptor (IL-10R1) and at least one constitutive promoter driving transcription of the IL-10 and IL-10R1 coding sequences.

13. An agent for the use of any one of claims 1-11 or the single expression vector of claim 12, wherein the vector further comprises a plasmid backbone, a kanamycin resistance gene, CMV promoter, β-globin intron, growth hormone polyA, and 2 AAV2 ITRs, and a self-cleaving 2a peptide positioned between the coding sequences of the IL-10 peptide and the coding sequences of the IL-10R1 peptide.

14. An agent for the use of any one of claims 1-11 and 13 or the single expression vector of claim 12 or 13, wherein IL-10 comprises a mutation in a hinge region of IL-10, optionally wherein IL-10 comprises a mutation where a phenylalanine at position 129 of an IL-10 wildtype sequence has been replaced with serine, threonine, alanine, or cysteine, optionally wherein the phenylalanine at position 129 of a wildtype sequence has been replaced with serine.

15. An agent for the use of any one of claims 1-11, 13 and 14 or the single expression vector of any one of claims 12-14, wherein the constitutive promoter is selected from the group consisting of ubiquitin promoters, CMV promoters, β-actin promoters, histone H4 promoters, EF-Iα promoters, PGK genes controlled by RNA polymerase II, promoter elements controlled by RNA polymerase I, promoter elements controlled by RNA polymerase III, U6 promoters (e.g. U6-1, U6-8, U6-9), H1 promoter, 7SL promoter, human Y promoters (e.g. hY1, hY3, hY4 and hY5), human MRP-7-2 promoter, Adenovirus VA1 promoter, human tRNA promoters, and 5s ribosomal RNA promoters, optionally wherein the constitutive promoter is a CMV promoter.

16. The agent for the use of any one of claims 1-11 and 13-15 or the single expression vector of any one of claims 12-15, wherein the vector further comprises a marker gene.

17. The agent for the use of any one of claims 1-11 and 13-16 or the single expression vector of any one of claims 12-16, wherein the vector is a plasmid.

## Patentansprüche

1. Mittel zur Verwendung bei der Behandlung einer Entzündung in einem Individuum, wobei das Mittel ein oder mehrere bakterielle, virale, Phagen-, Cosmid- oder künstliche Chromosomen-Vektoren ist, die Interleukin 10 (IL-10) und Interleukin-10-Typ-1-Rezeptor (IL-10R1) in antigenpräsentierenden Zellen in dem Individuum exprimieren, und wobei zumindest ein konstitutiver Promotor die Transkription der IL-10- und IL-10R1-Kodierungssequenzen antreibt.

2. Mittel zur Verwendung nach Anspruch 1, wobei die Verwendung die Herunterregulierung der IL-10-Signalisierung überwindet.

3. Mittel zur Verwendung nach Anspruch 1 oder 2, wobei die Verwendung eine konstitutive autokrine Signalisierung auf antigenpräsentierenden (IL-10R2-positiven) Zellen erzielt.

4. Mittel zur Verwendung nach einem der Ansprüche 1 bis 3, wobei IL-10 und IL-10R1 von einem einzigen Vektor exprimiert werden.

5. Mittel zur Verwendung nach einem der Ansprüche 1 bis 4, wobei der Vektor ein viraler Vektor ist, wobei der virale Vektor gegebenenfalls ein Vektor eines adenoassoziierten Virus oder ein Lentivirusvektor ist.

6. Mittel zur Verwendung nach einem der Ansprüche 1 bis 5, wobei die IL-10- und IL-10R1-Kodierungssequenzen als einzelne mRNA transkribiert werden.

7. Mittel zur Verwendung nach einem der Ansprüche 1 bis 6, wobei der Vektor weiters Folgendes umfasst:
(i) eine Kodierungssequenz für eine interne Ribosomeneintrittsstelle zwischen der IL-10- und der IL-10R1-Kodierungssequenz; oder
(ii) eine Kodierungssequenz für ein selbstspaltendes 2a-Peptid zwischen der IL-10und IL-10R1-Kodierungssequenz.

8. Mittel zur Verwendung nach einem der Ansprüche 1 bis 7, wobei die Entzündung durch Folgendes verursacht wird:
(i) neuropathische oder chronische Schmerzen und wobei der eine oder die mehreren Vektoren durch intrathekale Injektion zugeführt werden;
(ii) multiple Sklerose (MS) und wobei der eine oder die mehreren Vektoren durch intrathekale Injektion zugeführt werden;
(iii) eine Autoimmunerkrankung und wobei der eine oder die mehreren Vektoren durch intrathekale Injektion zugeführt werden; oder
(iv) multiple Sklerose (MS), chronische Schmerzen, Gelenksentzündung, Nervenentzündung oder eine Autoimmunkrankheit.

9. Mittel zur Verwendung nach einem der Ansprüche 1 bis 8, wobei sich die Entzündung in einem Gelenk befindet, wobei die Entzündung gegebenenfalls eine Nervenentzündung ist, und wobei der eine oder die mehreren Vektoren durch intraartikuläre Injektion zugeführt werden.

10. Mittel zur Verwendung nach einem der Ansprüche 1 bis 9, wobei die antigenpräsentierenden Zellen aus der aus Monoblasten, Monozyten, Astrozyten, Oligodendrozyten, Mikroglia, Makrophagen, B-Zellen, dendritischen Zellen, Schaumzellen, Lymphoblasten und B-Lymphozyten bestehenden Gruppe ausgewählt sind.

11. Mittel zur Verwendung nach einem der Ansprüche 1 bis 10, wobei die antigenpräsentierenden Zellen:
(i) aus einem zu behandelnden Individuum entnommen werden, mit einem oder mehreren Vektoren in vitro transduziert und wieder an das Individuum verabreicht werden; oder
(ii) stabil mit dem einen oder mehreren Vektoren transformiert und in Kultur gehalten werden.

12. Einzelner viraler oder bakterieller Expressionsvektor, umfassend die Kodierungssequenzen für Interleukin 10 (IL-10) und Interleukin-10-Typ-1-Rezeptor (IL-10R1) und zumindest einen konstitutiven Promotor, der die Transkription der IL-10- und IL-10R1-Kodierungssequenzen antreibt.

13. Mittel zur Verwendung nach einem der Ansprüche 1 bis 11 oder einzelner Expressionsvektor nach Anspruch 12, wobei der Vektor weiters eine Plasmid-Hauptkette, ein Kanamycin-Resistenzgen, einen CMV-Promotor, ein β-Globin-Intron, Wachstumshormon PolyA und 2 AAV-ITRs und ein selbstspaltendes 2a-Peptid, das zwischen den Kodierungssequenzen des IL-10-Peptids und den Kodierungssequenzen des IL-10R1-Peptids angeordnet ist, umfasst.

14. Mittel zur Verwendung nach einem der Ansprüche 1 bis 11 und 13 oder einzelner Expressionsvektor nach Anspruch 12 oder 13, wobei IL-10 eine Mutation in einer Gelenksregion von IL-10 umfasst, wobei IL-10 gegebenenfalls eine Mutation umfasst, wo ein Phenylalanin an Position 129 einer IL-10-Wildtypsequenz durch Serin, Threonin, Alanin oder Cystein ersetzt wurde, wobei das Phenylalanin an Position 129 einer Wildtypsequenz gegebenenfalls durch Serin ersetzt wurde.

15. Mittel zur Verwendung nach einem der Ansprüche 1 bis 11, 13 und 14 oder einzelner Expressionsvektor nach einem der Ansprüche 12 bis 14, wobei der konstitutive Promotor aus der aus Ubiquitin-Promotoren, CMV-Promotoren, β-Actin-Promotoren, Histon-H4-Promotoren, EF-Iα-Promotoren, PGK-Genen, die durch RNA-Polymerase II kontrolliert werden, Promotorelementen, die durch RNA-Polymerase I kontrolliert werden, Promotorelementen, die durch RNA-Polymerase III kontrolliert werden, U6-Promotoren (z. B. U6-1, U6-8, U6-9), H1-Promotor, 7SL-Promotor, menschlichen Y-Promotoren (z. B. hY1, hY3, hY4 und hY5), menschlichem MRP-7-2-Promotor, Adenovirus-VA1-Promotor, menschlichen tRNA-Promotoren und 5s-Ribosomen-RNA-Promotoren bestehenden Gruppe ausgewählt ist, wobei der konstitutive Promotor gegebenenfalls ein CMV-Promotor ist.

16. Mittel zur Verwendung nach einem der Ansprüche 1 bis 11 und 13 bis 15 oder einzelner Expressionsvektor nach einem der Ansprüche 12 bis 15, wobei der Vektor weiters ein Markergen umfasst.

17. Mittel zur Verwendung nach einem der Ansprüche 1 bis 11 und 13 bis 16 oder einzelner Expressionsvektor nach einem der Ansprüche 12 bis 16, wobei der Vektor ein Plasmid ist.

## Revendications

1. Agent à utiliser dans le traitement d'une inflammation chez un sujet, dans lequel l'agent est un ou plusieurs vecteurs bactériens, viraux, phagiques, cosmides ou chromosomiques artificiels exprimant l'interleukine 10 (IL-10) et le récepteur de type 1 de l'interleukine 10 (IL-10R1) dans des cellules présentant un antigène chez le sujet, et dans lequel au moins un promoteur constitutif entraîne une transcription des séquences de codage pour IL-10 et de IL-10R1.

2. Agent à utiliser selon la revendication 1, dans lequel l'utilisation surmonte la régulation à la baisse de la signalisation d'IL-10.

3. Agent à utiliser selon la revendication 1 ou 2, dans lequel l'utilisation permet d'obtenir une signalisation autocrine constitutive sur des cellules (IL-10R2-positives) présentant un antigène.

4. Agent à utiliser selon l'une quelconque des revendications 1 à 3, dans lequel l'IL-10 et l'IL-10R1 sont exprimées à partir d'un seul vecteur.

5. Agent à utiliser selon l'une quelconque des revendications 1 à 4, dans lequel le vecteur est un vecteur viral, facultativement dans lequel le vecteur viral est un vecteur de virus adéno-associé ou un vecteur de lentivirus.

6. Agent à utiliser selon l'une quelconque des revendications 1 à 5, dans lequel les séquences de codage pour IL-10 et IL-10R1 sont transcrites sous la forme d'un ARNm unique.

7. Agent à utiliser selon l'une quelconque des revendications 1 à 6, dans lequel le vecteur comprend en outre :
(i) une séquence de codage pour un site d'entrée de ribosome interne entre les séquences de codage pour IL-10 et IL-10R1 ; ou
(ii) une séquence de codage pour un peptide 2a auto-clivant entre les séquences de codage pour IL-10 et IL-10R1.

8. Agent à utiliser selon l'une quelconque des revendications 1 à 7, dans lequel l'inflammation est provoquée par :
(i) une douleur neuropathique ou chronique, et les un ou plusieurs vecteurs sont administrés par injection intrathécale ;
(ii) la sclérose en plaques (SEP), et les un ou plusieurs vecteurs sont administrés par injection intrathécale ;
(iii) une maladie auto-immune, et les un ou plusieurs vecteurs sont administrés par injection intrathécale ; ou
(iv) la sclérose en plaques (SEP), une douleur chronique, une inflammation articulaire, une neuroinflammation ou une maladie auto-immune.

9. Agent à utiliser selon l'une quelconque des revendications 1 à 8, dans lequel l'inflammation est située dans une articulation, facultativement dans lequel l'inflammation est une neuro-inflammation, et les un ou plusieurs vecteurs sont administrés par injection intraarticulaire.

10. Agent à utiliser selon l'une quelconque des revendications 1 à 9, dans lequel les cellules présentant un antigène sont choisies dans le groupe constitué de monoblastes, de monocytes, d'astrocytes, d'oligodendrocytes, de microglies, de macrophages, de cellules B, de cellules dendritiques, de cellules de mousse, de lymphoblastes et de lymphocytes B.

11. Agent à utiliser selon l'une quelconque des revendications 1 à 10, dans lequel les cellules présentant un antigène :
(i) sont retirées d'un sujet à traiter, transduites avec les un ou plusieurs vecteurs *in vitro,* et ré-administrées au sujet ; ou
(ii) sont transformées de manière stable avec les un ou plusieurs vecteurs et sont maintenues en culture.

12. Vecteur d'expression viral ou bactérien unique comprenant les séquences de codage pour l'interleukine 10 (IL-10) et le récepteur de type 1 de l'interleukine 10 (IL-10R1) et au moins un promoteur constitutif entraînant la transcription des séquences de codage pour IL-10 et IL-10R1.

13. Agent à utiliser selon l'une quelconque des revendications 1 à 11 ou vecteur d'expression unique selon la revendication 12, dans lequel le vecteur comprend en outre un squelette plasmidique, un gène de résistance à la kanamycine, un promoteur de CMV, un intron de β-globine, un polyA d'hormone de croissance et 2 ITR d'AAV2, et un peptide 2a auto-clivant positionné entre les séquences de codage du peptide d'IL-10 et les séquences de codage du peptide d'IL-10R1.

14. Agent à utiliser selon l'une quelconque des revendications 1 à 11 et 13 ou vecteur d'expression unique selon la revendication 12 ou 13, dans lequel l'IL-10 comprend une mutation dans une région charnière d'IL-10, facultativement dans lequel l'IL-10 comprend une mutation où une phénylalanine en position 129 d'une séquence de type sauvage d'IL-10 a été remplacée par de la sérine, de la thréonine, de l'alanine ou de la cystéine, facultativement dans lequel la phénylalanine en position 129 d'une séquence de type sauvage a été remplacée par de la sérine.

15. Agent à utiliser selon l'une quelconque des revendications 1 à 11, 13 et 14 ou vecteur d'expression unique selon l'une quelconque des revendications 12 à 14, dans lequel le promoteur constitutif est choisi dans le groupe constitué de promoteurs d'ubiquitine, de promoteurs de CMV, de promoteurs de β-actine, de promoteurs d'histone H4, de promoteurs d'EF-1α, de gènes PGK régulés par l'ARN polymérase II, d'éléments promoteurs régulés par l'ARN polymérase I, d'éléments promoteurs régulés par l'ARN polymérase III, de promoteurs U6 (par exemple U6-1, U6-8, U6-9), de promoteur de H1, de promoteur 7SL, de promoteurs de Y humains (par exemple hY1, hY3, hY4 et hY5), de promoteur de MRP-7-2 humain, de promoteur d'adénovirus VA1, de promoteurs d'ARNt humain, et de promoteurs d'ARN ribosomique 5S, facultativement dans lequel le promoteur constitutif est un promoteur de CMV.

16. Agent à utiliser selon l'une quelconque des revendications 1 à 11 et 13 à 15 ou vecteur d'expression unique selon l'une quelconque des revendications 12 à 15, dans lequel le vecteur comprend en outre un gène marqueur.

17. Agent à utiliser selon l'une quelconque des revendications 1 à 11 et 13 à 16 ou vecteur d'expression unique selon l'une quelconque des revendications 12 à 16, dans lequel le vecteur est un plasmide.
